# EUROPEAN PATENT SPECIFICATION

(11) **EP 0 876 826 B1**
(45) Date of publication and mention of the grant of the patent: **11.08.2004**
(21) Application number: 98107140.0
(22) Date of filing: 20.04.1998
(51) Int. Cl.: A61N 1/05, H01B 13/08, H01B 5/04, A61N 1/372, A61B 17/32

(54) **Implantable helical winding**
Implantierbare Spiralwicklung
Formation d'un câble hélicoidal implantable

(30) Priority: 07.05.1997 SE 9701719
(43) Date of publication of application: 11.11.1998
(73) Proprietor: St. Jude Medical AB, 175 84 Järfälla (SE)
(72) Inventor: Jarl, Per, 175 63 Järfälla (SE)

(56) References cited:
- EP-A- 0 092 798
- US-A- 3 946 727
- US-A- 5 362 113

## Description

### Technical field

The present invention relates to a to an electrode lead for a heart stimulator in accordance with the preamble of the independent claim.

### Background art

A helical winding as defined above is previously known from e.g. US-A-5,423,881, which shows a medical electrical lead comprising a conducting wire wound in a helical configuration. The lead is intended for use for endocardial stimulation by an implantable heart pacemaker and includes an electrode at the distal end of the lead and a connector at the proximal end.

This kind of winding has to be flexible to make introduction into e.g. a vein of a patient possible and a problem with such flexible windings is that a specified length of the winding cannot be maintained during implantation and explantation. Especially in connection with explantation the winding is easily extended. Another disadvantage of this type of winding is a low torsion stiffness.

In US-A-5,456,707 a pacing lead in the form of a helical winding is shown, the torsion characteristics of which are improved by providing an increased stiffness of an intermediate length of the lead. This increased stiffness is obtained by shrinking a tubing of Teflon over the intermediate section of the lead winding, or by a suitable coating sprayed onto the winding to restrict its flexibility, or by a tube which is interference fit between the inner diameter of the lead outer casing. This tube has a thickness so as to provide minimum clearance with the winding to prevent outward expansion of the winding, i.e. to increase the stiffness of the winding. These special measures for increasing the stiffness of the winding, however, complicate the manufacture of the lead and raises its price.

EP-A1-0 092 798 shows a multipole coaxial circuit intended to be used as an electrode for electric stimulation of body tissue. The circuit comprises at least one conductor in the form of a helically wound metal band for reducing the diameter of the lead and improving its flexibility. However, neither this construction solves the problems of extension of the winding, especially in connection with explantation of the lead, and poor torsion stiffness.

It is therefore a main object of the present invention to provide a helical winding, which is flexible enough to make implantation into a patient's body possible as well as explantation therefrom, the elongation of which can be reliably specified, and which is torsion stable.

### Disclosure of the invention

This object is accomplished by an electrode lead of the kind defined in the preamble of claim 1 characterized in that adjacent turns of the winding of the lead are partially overlapping each other and in that said band is formed such that overlapping portions of the band are engaging each other to limit the relative axial motion of adjacent turns. Thus a certain relative mobility is allowed between adjacent turns of the winding according to the invention to give the winding the necessary flexibility, however, as overlapping portions of the band are provided with engaging means which limit the relative motion of adjacent turns, the length of the winding can only vary within specified limits, i.e. the elongation of the winding can be specified. The torsion stiffness is also improved and this stiffness is successively increased when rotating the winding in one specific direction, since the turns are partially overlapping.

According to advantageous embodiment of the winding according to the invention the cross-section of the band has the shape of two V-forms oppositely directed away from the general plane through the band, one of the shanks of each V-form being formed of a common mid-portion of the band. In this embodiment the flexibility of the winding is a result of the flexibility of the material of the band and possibly the result of a minor relative sliding of the overlapping portions.

According to the invention the band is made of an electrically conducting material and is used as a conductor of an electrode lead for a heart stimulator. For this application the winding is normally enclosed in an outer casing. A winding according to the invention made of a band of electrically conducting material can also be used as an effective shield against electromagnetic disturbances for a conductor extending inside the winding.

### Brief description of the drawings

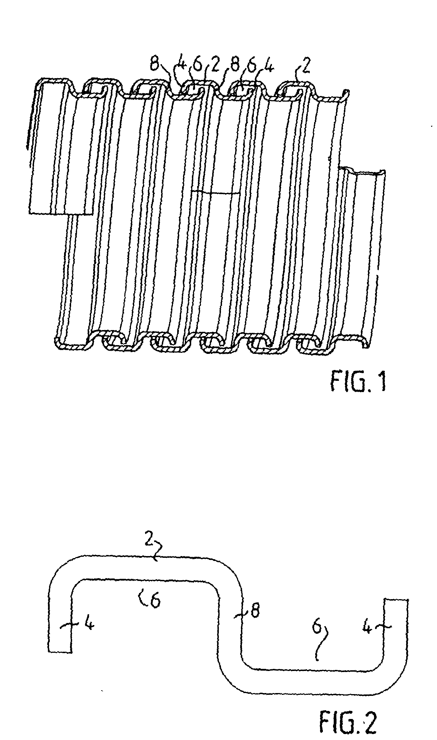

To explain the invention more in detail embodiments chosen as examples will now be described with reference to the drawings, on which figure 1 shows a longitudinal section through a portion of a winding according to the invention, and figures 2-6 show different cross-sectional shapes which can be used for the winding according to the invention.

### Description of preferred embodiments

Figure 1 shows a longitudinal section through a winding in an electrode lead according to the invention, wound of a band 2 having an S-shaped cross-section, cf. figure 2. Adjacent turns of the winding are partially overlapping, such that the upstanding edge portions 4 of the S-shaped band 2 of one turn are positioned inside the "valleys" 6 inside the corresponding edge portions 4 of adjacent turns. In this configuration adjacent turns can be moved in the axial direction of the winding relative each other a distance corresponding to the width of the valley 6, which is limited by an edge portion and an upstanding mid portion 8 of the turn in question. This relative mobility of the winding turns make the winding flexible and compression and elongation respectively of the winding is limited by the engagement of an edge portion 4 with a mid-portion 8 and engagement with an edge portion 4 respectively of adjacent turns in the winding.

The winding is wound of an electrically conducting material, like a metal, and the winding can be used as a pacing lead for the connection of a pacemaker to a patient's heart. The winding is then preferably enclosed in an outer casing of isolating material.

Such an electrically conducting winding can also be used as a shield against electromagnetic disturbances for a conductor extending inside the winding. Such a protection against disturbance signals is of importance for certain applications.

The winding according to the invention also exhibits an increased torsion stiffness and since the winding is formed of partially overlapping turns this torsion stiffness is successively increased when rotating the winding in one specific direction.

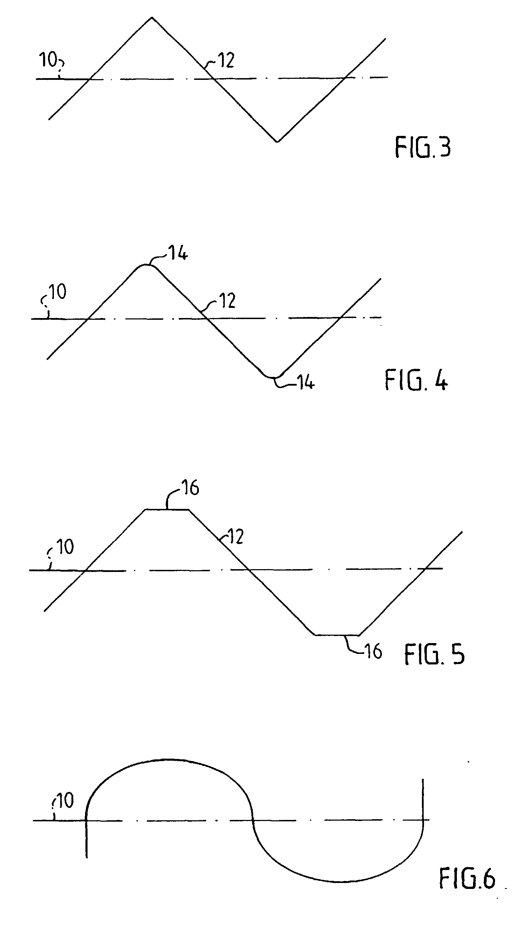

In figure 3-6 alternative cross-sectional shapes of the winding band are shown.

Thus figure 3 shows a saw-toothed cross-sectional shape of the band comprising to V-forms oppositely directed away from the general plane through the band. One of the shanks of each V-form is formed of a common mid portion 12 of the band. In this embodiment the flexibility of the winding is obtained as a result of the flexibility in the material of the band and possibly, to a minor sliding of the overlapping portions of adjacent turns of the winding relative each other.

Figures 4 and 5 show variations of the band shape in figure 3 with rounded tops 14 of the V-forms and with the tops of the V-forms cut parallel to the general plane 10 through the band respectively.

In figure 6 still another alternative of the cross-sectional shape of the band is shown. In this embodiment the cross-section of the band exhibits a sinusoidal elliptic shape. Obviously also other cross-sectional shapes of the

band are possible, like e.g. an ordinary sinusoidal shape. The only condition which has to be fulfilled is that the cross-sectional shape of the band must be such that overlapping portions of the band are engaging each other to limit the relative axial motion of adjacent turns of the winding.

When manufacturing the winding according to the invention a band or a flat wire of a suitable material, like a metal, is formed to the desired cross-sectional shape with longitudinally extending engagement means as specified below. The band is then wound on a mandrel to a winding of desired axial length and the mandrel is removed out of the winding. The band is wound with adjacent turns partially overlapping each other and the engagement means are formed such that they are engaging overlapping portions of the band to limit the relative axial motion of adjacent turns of the manufactured winding.

After terminated winding the winding can be enclosed in an outer casing of e.g. a polymeric material.

## Claims

1. An electrode lead for a heart stimulator comprising a helical winding, wound of a band (2) being made of an electrically conducting material to a length considerably exceeding its diameter, said lead being adapted for implantation into a patient's body, **characterized in that** adjacent turns of the winding are partially overlapping each other and **in that** said band (2) is formed such that overlapping portions of the band are engaging each other to limit the relative axial motion of adjacent turns, said winding being provided with an electrode pole at a distal end portion and a connector at the proximal end for connection to a pulse generator.

2. The electrode lead for a heart stimulator according to claim 1, **characterized in that** said band is formed such that maximum allowable axial motion of adjacent winding turns is less than half the width of the band (2).

3. The electrode lead for a heart stimulator according to claims 1 or 2, **characterized in that** said band (2) is formed as two longitudinal channels, each one having one of its sides open, said open sides being turned in opposite directions away from the general plane (10) through the band.

4. The electrode lead for a heart stimulator according to claim 3, **characterized in that** the cross-section of the band (2) has the shape of two U-forms oppositely directed away from the general plane (10) through the band, one of the shanks of each U-form being formed of a comman mid-portion (8) of the band.

5. The electrode lead for a heart stimulator according to claim 3, **characterized in that** the cross-section of the band is S-shaped.

6. The electrode lead for a heart stimulator according to claim 3, **characterized in that** the cross-section of the band has the shape of two V-forms oppositely directed away from the general plane through the band, one of the shanks of each V-form being formed of a comman mid-portion (12) of the band.

7. The electrode lead for a heart stimulator according to claim 6, **characterized in that** the tops (14) of the V-forms are rounded.

8. The electrode lead for a heart stimulator according to claim 6, **characterized in that** the tops (16) of the V-forms are plane.

9. The electrode lead for a heart stimulator according to claim 3, **characterized in that** the cross-section of the band has a sinusoidal shape.

10. The electrode lead for a heart stimulator according to claim 9, **characterized in that** the cross-section of the band has a sinusoidal elliptic shape.

11. An electrode lead for a heart stimulator comprising a winding according to any one of the claims 1 through 10, the winding being disposed as a shield around a conductor extending inside the winding.

## Patentansprüche

1. Elektrodenleitung für einen Herzstimulator, enthaltend eine wendelförmige Wicklung die aus einem Band (2) aus elektrisch leitendem Material bis zu einer Länge gewickelt ist, die wesentlich ihren Durchmesser überschreitet, wobei die genannte Leitung für die Implantation in den Körper eines Patienten geeignet ist, **dadurch gekennzeichnet, dass** benachbarte Windungen der Wicklung einander teilweise überlappen und dass das genannte Band (2) so ausgebildet ist, dass überlappende Teile des Bandes ineinander greifen, um die relative Axialbewegung benachbarter Windungen zu begrenzen, die genannte Wicklung an einem distalen Endabschnitt mit einem Elektrodenpol und am proximalen Ende mit einem Verbinder zur Verbindung mit einem Impulsgenerator versehen ist.

2. Elektrodenleitung für einen Herzstimulator nach Anspruch 1, **dadurch gekennzeichnet, dass** das genannte Band derart ausgebildet ist, dass die maximal zulässige axiale Bewegung benachbarter Wicklungswindungen kleiner als die Hälfte der Breite des Bandes (2) ist.

3. Elektrodenleitung für einen Herzstimulator nach den Ansprüchen 1 oder 2, **dadurch gekennzeichnet, dass** das genannte Band (2) in Form von zwei Längskanälen ausgebildet ist, von denen jeder eine seiner Seiten offen hat, die genannten offenen Seiten in entgegengesetzten Richtungen von der Hauptebene (10) durch das Band abgewendet sind.

4. Elektrodenleitung für einen Herzstimulator nach Anspruch 3, **dadurch gekennzeichnet, dass** der Querschnitt des Bandes (2) die Gestalt zweier U-Formen aufweist, die von der Hauptebene (10) durch das Band entgegengesetzt weggerichtet sind, wobei einer der Schenkel jeder U-Form als ein gemeinsamer Mittelteil (8) des Bandes ausgebildet ist.

5. Elektrodenleitung für einen Herzstimulator nach Anspruch 3, **dadurch gekennzeichnet, dass** der Querschnitt des Bandes S-förmig ist.

6. Elektrodenleitung für einen Herzstimulator nach Anspruch 3, **dadurch gekennzeichnet, dass** der Querschnitt des Bandes die Gestalt zweier V-Formen aufweist, die von der Hauptebene durch das Band entgegengesetzt weggerichtet sind, wobei einer der Schenkel jeder V-Form als ein gemeinsamer Mittelteil (12) des Bandes ausgebildet ist.

7. Elektrodenleitung für einen Herzstimulator nach Anspruch 6, **dadurch gekennzeichnet, dass** die Spitzen (14) der V-Formen abgerundet sind.

8. Elektrodenleitung für einen Herzstimulator nach Anspruch 6, **dadurch gekennzeichnet, dass** die Spitzen (16) der V-Formen eben sind.

9. Elektrodenleitung für einen Herzstimulator nach Anspruch 3, **dadurch gekennzeichnet, dass** der Querschnitt des Bandes eine Sinusform aufweist.

10. Elektrodenleitung für einen Herzstimulator nach Anspruch 9, **dadurch gekennzeichnet, dass** der Querschnitt des Bandes eine elliptische Sinusform aufweist.

11. Elektrodenleitung für einen Herzstimulator enthaltend eine Wicklung nach einem der Ansprüche 1 bis 10, wobei die Wicklung als ein Schild um einen sich innerhalb der Wicklung erstreckenden Leiter angeordnet ist.

## Revendications

1. Câble d'électrode destiné à un stimulateur cardiaque comprenant un enroulement hélicoïdal, consistant en une bande (2), constituée d'un matériau électroconducteur, enroulée sur une longueur dépassant considérablement son diamètre, le câble étant conçu pour une implantation dans le corps d'un patient, **caractérisé en ce que** des spires voisines de l'enroulement se chevauchent partiellement les unes les autres et **en ce que** la bande (2) est formée de telle sorte que des parties de recouvrement de la bande coopèrent les unes avec les autres pour limiter le mouvement axial relatif de spires voisines, l'enroulement étant muni d'un pôle d'électrode à une partie d'extrémité distale et d'un connecteur à l'extrémité proximale, en vue de la connexion à un générateur d'impulsions.

2. Câble d'électrode destiné à un stimulateur cardiaque suivant la revendication 1, **caractérisé en ce que** la bande est formée de telle sorte qu'un mouvement axial maximum admissible de spires d'enroulement voisines est inférieur à la moitié de la largeur de la bande (2).

3. Câble d'électrode destiné à un stimulateur cardiaque, suivant la revendication 1 ou 2, **caractérisé en ce que** la bande (2) est formée en tant que deux canaux longitudinaux ayant chacun l'un de ses côtés ouvert, les côtés ouverts étant tournés dans des sens opposés s'écartant du plan (10) général, d'un bout à l'autre de la bande.

4. Câble d'électrode destiné à un stimulateur cardiaque, suivant la revendication 3, **caractérisé en ce que** la coupe transversale de la bande (2) a la forme de deux U orientés de façon opposée, à l'écart du plan (10) général, d'un bout à l'autre de la bande, l'une des jambes de chaque U étant constituée d'une partie (8) intermédiaire commune de la bande.

5. Câble d'électrode destiné à un stimulateur cardiaque, suivant la revendication 3, **caractérisé en ce que** la section transversale de la bande a une forme de S.

6. Câble d'électrode destiné à un stimulateur cardiaque, suivant la revendication 3, **caractérisé en ce que** la section transversale de la bande a la forme de deux V orientés de façon opposée, à l'écart du plan général, d'un bout à l'autre de la bande, l'une des jambes de chaque V étant constituée d'une partie (12) intermédiaire commune de la bande.

7. Câble d'électrode destiné à un stimulateur cardiaque, suivant la revendication 6, **caractérisé en ce que** les sommets (14) des V sont arrondis.

8. Câble d'électrode destiné à un stimulateur cardiaque, suivant la revendication 6, **caractérisé en ce que** les sommets (16) des V sont plans.

9. Câble d'électrode destiné à un stimulateur cardiaque, suivant la revendication 3, **caractérisé en ce que** la coupe transversale de la bande a une forme sinusoïdale.

10. Câble d'électrode destiné à un stimulateur cardiaque, suivant la revendication 9, **caractérisé en ce que** la coupe transversale de la bande a une forme elliptique sinusoïdale.

11. Câble d'électrode destiné à un stimulateur cardiaque comprenant un enroulement, suivant l'une quelconque des revendications 1 à 10, l'enroulement étant disposé en tant que blindage autour d'un conducteur s'étendant à l'intérieur de l'enroulement.
